# EUROPEAN PATENT SPECIFICATION

(11) **EP 2 007 471 B1**
(45) Date of publication and mention of the grant of the patent: **08.05.2019**
(21) Application number: 06802612.9
(22) Date of filing: 30.08.2006
(51) Int. Cl.: A61M 37/00, A61M 13/00, A61B 18/00, A61B 17/34

(54) **AUTOMATIC SMOKE EVACUATOR AND INSUFFLATION SYSTEM FOR SURGICAL PROCEDURES**
AUTOMATISCHER RAUCHEVAKUATOR UND INSUFFLATIONSSYSTEM FÜR CHIRURGISCHE EINGRIFFE
SYSTÈME D'INSUFFLATION ET D'ÉVACUATION AUTOMATIQUE DE FUMÉE POUR PROCÉDURES CHIRURGICALES

(30) Priority: 20.04.2006 US 379406
(43) Date of publication of application: 31.12.2008
(73) Proprietor: I.C. Medical, Inc., Phoenix, AZ 85023 (US)
(72) Inventor: COSMESCU, Ioan, Phoenix, AZ 85023 (US)
(74) Representative: Hanna Moore + Curley
(86) International application number: PCT/US2006/033838
(87) International publication number: WO 2007/123565

(56) References cited:
- US-A- 4 207 887
- US-A- 4 735 603
- US-A- 4 735 603
- US-A- 5 006 109
- US-A- 5 098 375
- US-A- 5 098 375
- US-A- 5 139 478
- US-A- 5 199 944
- US-A- 5 246 419
- US-A- 5 246 419
- US-A1- 2006 052 661

## Description

### FIELD OF INVENTION

The present invention generally relates to an automatic smoke evacuator system for surgical procedures having a means for the insufflation of the abdominal cavity; and replacement of gas which is removed from the abdominal cavity as a result of the smoke evacuation which is performed during the surgical procedures. More specifically, the present invention relates to an automatic smoke evacuator having insufflator means wherein the insufflator means initially fills the intra-abdominal cavity with a desired pressure and then replaces the gas removed by the smoke evacuator at the same flow rate and at the same time at which the smoke evacuator is activated.

### BACKGROUND OF THE INVENTION

The apparatus described in the U.S. Patent No. 5,199,944 can remove a precise volume of gas, along with the smoke, from the abdominal cavity during surgical procedures. The flow of the gas removed can be adjusted precisely and is determined by the potential of the insufflator which is used. The insufflator can quickly and efficiently replace the same volume of gas that is removed with the smoke evacuator.

While this design works very well in normal conditions, the efficiency in many instances where the insufflator has a low flow rate is diminished since the smoke evacuator flow has to be very low in order to maintain the intra-abdominal pressure. Another inconvenience results from the fact that the insufflators available on the market react to the drop in pressure associated with the gas that is removed by the smoke evacuator; and to the leads which activate and insufflate the gas at a lower rate when the difference between the adjusted pressure and the peritoneum pressure is small. Accordingly, the insufflator in the prior art needed a larger drop in pressure in order for the flow to increase and be useful. However; a large drop in pressure would result in the collapse of the peritoneum which would be very dangerous when performing the surgery.

The insufflators currently available on the market deliver an intermittent flow of gas because the pressure in the peritoneum can not be measured while the gas is flowing through the tubing. Further, this intermittent flow is inconvenient because the suction from the smoke evacuator is continuous.

Even with all the previously described inconveniences, the smoke evacuation performed by the automatic smoke evacuator described in U.S. Patent No. 5,199,944 was very helpful, relatively efficient, and more superior to all the laparoscopic smoke evacuators existing on the market at the time. Nevertheless, improvements to the design are necessary to overcome some of the inconveniences described and these improvements are the subject of this invention.

In this invention a new and improved smoke evacuator is described having an increased efficiency and many patient safety features. The smoke evacuator presented in this invention can safely and efficiently remove the insufflation gas from the peritoneum. This will reduce or eliminate the pain and discomfort associated with laparoscopic procedures since this pain and discomfort are due to the slow absorption of the CO2 gas left in the peritoneum by the patient tissue.

The smoke evacuator also has a vacuum sensor which will shut off the smoke evacuator if patient tissue is trapped in the instrument; and which will turn on the smoke evacuator when the tissue is released. It will also illuminate a "change filter" caution indicator when the filter is dirty and its efficiency is reduced.

The smoke evacuator also has a pressure sensor which will automatically turn on the smoke evacuator if the intra-abdominal pressure reaches unsafe limits. This is a very important feature because excessive pressure within the patient can cause an embolism which can be deadly. The new pressure sensor is used for the insufflation part of the apparatus to determine the intra-abdominal pressure when the smoke evacuator includes the insufflation means.

As described above; one of the deficiencies of the laparoscopic smoke evacuator described in U.S. Patent No. 5,199,944 was that the insufflators could not always keep up with the smoke evacuation. In the design of the present invention, that problem is completely solved by having an insufflator added to the smoke evacuator which, besides initially insufflating the peritoneum with the desired pressure, will automatically replace the volume of gas eliminated by the smoke evacuator at exactly the same time with exactly the same flow so that the peritoneum will remain with the same pressure that has been chosen by the surgeon. In order for the combination smoke evacuator/insufflator to work correctly the smoke evacuator has to be completely automatic. In other words, the smoke evacuator has to start only when the surgical device laser or ESU is activated, and at the same time deactivate shortly after the surgical device is deactivated. Otherwise, if the smoke evacuator functions continuously, the insufflator must continuously replace the gas which will result in excessive gas waste and an operating room that will become filled with that gas, which is typically CO₂.

Also, in the past, recirculators were used and attached to the insufflator in order to eliminate the smoke. Recirculators suction gas from the abdomen, filter the smoke, and return it to the abdomen. However, recirculators present many deficiencies and are prone to creating excessive pressure conditions within the patient's body. None of the safety features described above with respect to the present invention are found in recirculators.

The present state of the art is represented by US 5,098,375 A, US 5,246,419 A, US 4,735,603 A, US 5,006,109 A, US 4,207,887 A, and US 2006/052661 A1.

### SUMMARY OF THE INVENTION

The present invention provides an automatic smoke evacuation and insufflation apparatus as detailed in claim 1. Advantageous features are provided in the dependent claims. The present invention provides a smoke evacuator for laparoscopic procedures with an adjustable and precise flow and with a solenoid valve that will open only when the smoke evacuator is activated, in accordance with claims which follow. The solenoid valve is normally closed so that no gas will escape. This is necessary so the intra-abdominal pressure can be easily maintained.

Another object of the present disclosure is to provide a smoke evacuator with a pressure sensor for monitoring the intra-abdominal pressure. If the intra-abdominal pressure exceeds a certain preselected level, the smoke evacuator automatically turns on thereby reducing the pressure to a safe level. The same pressure sensor is used to caution the staff if the patient is not attached to the smoke evacuator. The sensor also can be used for monitoring the intra-abdominal pressure for the insufflator if a separate insufflator is attached to the smoke evacuator.

It is still another object of the present disclosure to provide a smoke evacuator with a vacuum sensor which will turn the smoke evacuator off if the instrument becomes obstructed or occluded with tissue or other elements.

It is yet another object of the present disclosure to provide a smoke evacuator with an insufflation potential which will initially safely insufflate the abdominal cavity, and then monitor the pressure so it is maintained at a desired level by simultaneously using an insufflator to replace the volume of gas removed by the smoke evacuator at the same rate that the gas is being removed by the smoke evacuator.

Another object of the present disclosure is to provide a gas warmer for high flow rate. The warming temperature will depend on the gas flow rate and will be directly proportional with the flow rate. The higher the flow rate, the higher the temperature.

It is important to mention that while the smoke evacuator described in this disclosure can be, and is, used as a separate unit, the insufflator described in this disclosure cannot be used without being included in the smoke evacuator described in this disclosure since the pressure described in the smoke evacuator portion of the disclosure is also used as the main pressure sensor for the insufflator. Without this pressure sensor, the insufflator will not function.

It is still another object of the present disclosure to provide a high flow insufflator with improved patient safety features.

In accordance with one embodiment of this disclosure, an automatic smoke evacuator unit system and apparatus for open and endoscopic/laparoscopic procedures is disclosed comprising, in combination, a vacuum pump means for providing the necessary vacuum for removing the smoke and debris from the surgical site in open and/or laparoscopic procedures, and a pump control means for controlling the activation and deactivation of the pump when the surgical device is activated.

The pump control is connected with an off time delay mechanism which will keep the pump running several seconds after the surgical device has been deactivated. The time delay period for shut off can be adjusted by the adjusting pot. The OFF time delay is connected with a laser sensor which will activate the off time delay when the laser is activated. An ESU sensor will activate the off time delay when an ESU is activated. A manual switch bypasses the sensors and activates the smoke evacuator when the switch is on.

The automatic smoke evacuator system and apparatus also comprises a solenoid valve which will open the flow pass when the smoke evacuator is activated for open procedures that are conducted with a high flow rate. A second solenoid valve will open when the smoke evacuator is activated for laparoscopic procedures.

A flow sensor for laparoscopic flow will show the precise flow rate when the smoke evacuator is activated and will exhibit more functions when the smoke evacuator is used with the insufflator function. This will be explained in detail with reference to the second embodiment.

The automatic smoke evacuator system and apparatus of the present disclosure also comprises a pressure sensor connected to a first comparator that will activate a relay where the contact for the relay will close, sending a signal to activate the vacuum pump. This will only happen when the pressure measured by the pressure sensor exceeds the maximum limits that are set in association with the other side of the first comparator (this is the reference voltage).

A second comparator will identify when no pressure is present. The pressure sensor will have more functions when used with the insufflator presented in the second embodiment. A vacuum sensor is connected to one side of the second comparator and the output of this comparator is connected with an adjustable off time delay and the output of the off time delay is connected with a relay. The contacts of the relay will turn off the vacuum pump and close the solenoid valve if the vacuum level is too high and an occlusion is present. The other side of the second comparator is connected to the flow and vacuum level adjustor. This too will have more functions in the second embodiment.

A first filter means is also included for filtering the smoke and debris which is evacuated using the smoke evacuator, and a fluid trap is present for preventing the fluids from reaching the vacuum pump. The first filter is connected through tubes and through solenoids to one side of the vacuum pump. A second filter for filtering gases and odor is located on the output side of the vacuum pump.

In accordance with yet another embodiment of this disclosure an automatic smoke evacuator system and apparatus having an automatic insufflation means is presented which includes, in combination, an automatic smoke evacuator means identical with the smoke evacuator described in the first embodiment and an insufflator means which initially fills the intra-abdominal cavity with a desired pressure and then automatically replaces the gas removed by the smoke evacuator at the same flow rate and at the same time that the smoke evacuator is activated.

The insufflation means comprises a gas source under pressure, a high pressure filter means for stopping particles and bacteria present in the gas tank, a high pressure sensor which will measure the volume of gas existing in the gas tank, and a pressure regulator for dropping the pressure to a working pressure. Further, the gas will pass through a safety solenoid. The safety solenoid will shut off the gas flow if the patient's intra-abdominal pressure exceeds the adjusted maximum pressure.

The automatic smoke evacuator system and apparatus having an automatic insufflation means of the present disclosure further comprises a mechanical release valve which will let the gas escape if the gas pressure exceeds the pressure adjusted at the pressure regulator. This safety measure is for protecting the patient against an increase in the working pressure in the event of a failure with the pressure regulator or a failure with the adjustor for the pressure regulator. From the mechanical release valve, the gas will pass through a proportioned solenoid valve. The proportioned solenoid valve will adjust the precise flow rate delivered to the patient when adjusted by the flow adjust or by the flow rate of the smoke evacuator flow meter. Accordingly, when the smoke evacuator is activated, the insufflator will simultaneously deliver gas at the exact flow rate at which the smoke evacuator is removing gas, smoke, and debris in order to efficiently replace the gas removed by the smoke evacuator.

The proportioned solenoid valve will also have a role in safety by shutting completely off when the pressure exceeds the desired adjusted pressure or if the insufflator flow is higher than the smoke evacuator flow. Further, an exhaust solenoid valve will let the gas escape if the pressure rises over the desired predetermined pressure, or if the flow of the insufflator is higher than the flow of smoke evacuator. The solenoid therefore has the role of balancing the pressure. This exhaust solenoid valve will be the first to be activated and, only if the pressure is still rising, will the proportioned solenoid valve completely shut off. If the pressure is still rising, then the smoke evacuator is automatically activated so that gas is actively and rapidly removed in order to reduce the danger of embolism. This is a safety feature that is unique to this apparatus. Further in line is a flow meter which will display the real flow rate of the insufflator. Also, the flow rate measured by this flow meter is continuously compared with the flow meter readings from the smoke evacuator so that if the flow rate of the insufflator is higher than the flow rate of the smoke evacuator, the safety feature described above will immediately activate. The flow meter for the insufflator will also control the gas temperature by sending a signal proportional with the flow level to the gas warmer.

A patient pressure sensor will measure the patient's abdominal pressure only when a needle is used. It will also help to locate the position of the needle in the peritoneum. The pressure sensor on the insufflation line will have an active role in normal use only when there is no insufflation flow. This is due to the fact that while the gas is flowing through the insufflation tubing, the resistance in the line will create higher pressure giving a distorted reading of the real intra-abdominal pressure thereby turning the insufflation off because of excessive pressure even if there is no intra-abdominal pressure. In this situation, the active role of the pressure readings during the insufflation will be taken by the pressure sensor for the smoke evacuator. Therefore, the insufflation apparatus described in this application cannot be used as a stand alone unit; it can only be used in combination with the automatic smoke evacuator system and apparatus described as the first embodiment.

The present disclosure directed to the automatic smoke evacuator system and apparatus having an automatic insufflation means further comprises a gas warmer which is different than any previous gas warmer design because the temperature of the gas warmer is automatically adjusted by the gas flow rate and not by the exact temperature. The gas warmer turns ON and OFF to maintain that temperature. In existing gas warmer designs, the gas is warmed to a certain temperature regardless of the gas flow rate. However, that system is not adequate because the higher the flow rate of the gas, the cooler the gas will become when expended in the abdominal cavity. Therefore, if the temperature is adjusted too low in existing gas warmers, the drop in gas temperature will increase and the resulting low temperature could freeze the tissue. On the other hand, if the temperature is high enough for a high flow rate, then in a low flow area where the expansion of the gas is much lower, the gas will be too hot and will burn the patient because the drop in temperature will not be great enough.

In the gas warmer design of the present disclosure, the flow rate itself will adjust the gas temperature. The higher the flow rate, the higher the temperature of the gas warmer and the lower the flow rate, the lower the temperature of the gas warmer.

### BRIEF DESCRIPTION OF THE DRAWINGS

FIG. 1 represents a base schematic of a first embodiment of the automatic smoke evacuator and insufflation system and apparatus of the present invention. This embodiment is presented as a stand alone apparatus with special safety features.
FIG. 2 represents a base schematic of a second embodiment of the automatic smoke evacuator and insufflation system and apparatus of the present invention shown in FIG. 1 having an automatic insufflator means for maintaining the appropriate gas pressure within a patient's abdominal cavity.

### DETAILED DESCRIPTION

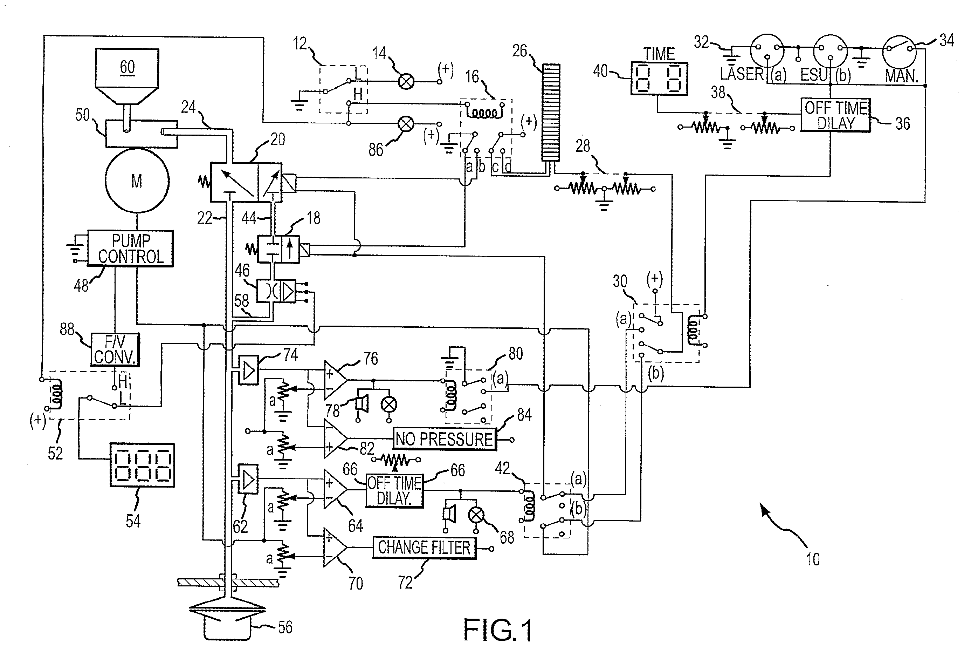

FIG. 1 depicts an automatic smoke evacuator and insufflation system and apparatus 10. The exact sequence of the system and apparatus set up and operation will be followed in the detailed description of this first preferred embodiment of the invention.

Starting with the set up of the system and apparatus, the unit is turned on at the ON switch 12 which can be set on high or low depending on whether the surgical procedure is open or laparoscopic. The ON switch 12 is set in the low position for laparoscopic procedures and the low light 14 will be illuminated. A first relay 16 is not activated so the contacts remain in the normal non-energized position and a ground signal is present at the first contact 16a. The ground signal is sent to the ground side of a first solenoid valve 18. A three way second solenoid valve 20 is normally closed to a first tubing 22 and normally open at a second tubing 24. The three way second solenoid valve 20 will stay this way and in this mode since the ground side of the second solenoid valve 20 is open as a result of the open second contact 16b of the first relay 16.

The third contact 16c of the first relay 16 is closed which results in a signal being sent to the bar graph 26 on the low flow rate side so that when the second part of the flow adjusting pot 28 is set to a certain flow rate, the bar graph 26 will reflect the flow rate at which the smoke evacuator will function. The first part of the double adjusting pot 28 will send the adjusted flow signal to the second contact 30b of the second relay 30.

The LASER and ESU sensors (not shown) will be connected to first and second contacts 32a, 32b, respectively, which are parallel to one another. The manual switch 34 can bypass the sensors thereby enabling the automatic smoke evacuator system and apparatus 10 to be activated manually. When a laser or an ESU unit is activated, its respective sensor will send an ON signal to the off time delay 36. The adjusting double pot 38 will adjust the off time delay 36 which is the time that the smoke evacuator will continue to work after the LASER or ESU unit is deactivated and the ON signal is removed. This is necessary to ensure that all of the smoke will reach the filter and that none of the smoke is trapped in the tubing. The second part of the adjusting double pot 38 will control a first display 40 which will display the adjusted delay time.

A signal is sent from the off time delay 36 to second relay 30 which is then activated. The second relay 30 has first and second contacts 30a, 30b which are normally open. When the second relay 30 is activated, the first and second contacts 30a, 30b will close and send a signal through first and second contacts 42a, 42b in third relay 42 which are normally closed. The signal will continue to both the first solenoid valve 18 and the second solenoid valve 20, but only the first solenoid valve 18 is activated because the ground is received from the first contact 16a of the first relay 16 and the second solenoid valve 20 has an open ground at the second contact 16b of the first relay 16.

By opening the first solenoid valve 18, the flow is opened through third tubing 44 and flow rate meter 46. At the same time, the second contact 30b of the second relay 30 will close sending the adjusted flow signal from the flow adjusting pot 28 through the normally closed first and second contacts 42a, 42b of the third relay 42, to the pump control 48 thereby turning on the pump 50 with the exact flow rate that has been adjusted by flow adjusting pot 28.

A fourth relay 52 has the contact (L) closed in the normal position so that the flow rate meter 46 will send the flow rate reading to a second display 54.

The flow line is now open through the low side for laparoscopic procedure. The direction of the flow is as follows:
When the pump is activated and the first and second solenoid valves 18,20 are open, a suction is applied at the filter 56 by the vacuum pump 50. Gas, which includes smoke, debris and sometimes accidentally fluids, is sucked from the patient through the tubing, and will reach the ULPA filter 56 which has a fluid trap for the fluids. The smoke is filtered by the filter 56 and the clean gases will pass through the first tubing 22, up to the Y connector 58, and then through the flow rate meter 46. The clean gases will continue to pass through the first solenoid valve 18, through the normally open side of the three way second solenoid valve 20 to pump 50, and then through the gas filter 60 which will filter the gasses and odor. This is how the first embodiment of the present invention functions during normal conditions in the laparoscopic mode.

If tissue is trapped, or any kind of occlusion condition appears, then the vacuum sensor 62 will detect it and send a signal to the first comparator 64 if the occlusion or obstruction condition level is higher than the limit adjusted by adjusting pot 64a (the reference value). The first comparator 64 will send an ON signal to the second off time delay 66, and from the off time delay 66 the signal is then sent to an audio and visual alarm 68 and to the third relay 42 which will open the normally closed contacts 42a, 42b thereby stopping the pump 50 and closing the second solenoid valve 20.

The same vacuum sensor 62 will send a signal to a second comparator 70 which has the adjusting pot 70b reference adjusted for a lower vacuum level. This will turn on a "change filter" light 72 when the resistance in the filter 56 increases as a result of filling up with smoke particles. This ensures that the filter 56 will not continue to be used if the efficiency is dropping. If the filter 56 is not changed after becoming full, the system and apparatus 10 will shut off shortly afterwards because the resistance in the filter 56 will reach a higher level which will be read as an occlusion.

If the intra-abdominal pressure is too high, then the pressure sensor 74 will send a signal to the third comparator 76. If the pressure is above the safe pressure limit adjusted by the adjusting pot 76a (the reference value), then an excessive pressure signal is sent to the second audio and visual alarm 78 and to a fifth relay 80 so that the normally open contact 80a will close and send an ON signal to off time delay 36. The pump 50 will turn ON and the second solenoid valve 20 will open so the gas from the intra-abdominal cavity will be allowed to be suctioned until the pressure is brought to safe levels.

The same pressure sensor 74 is used to sense whether the tubing is attached to the patient since the pressure will be zero if the patient tubing is not attached. When the tubing is attached to the patient, pressure is present which will be read by the pressure sensor 74 which will send a signal to a fourth comparator 82. The minimum pressure is adjusted by the adjusting pot 82a. When the pressure is zero, the no pressure light 84 is on. When pressure sensed by the pressure sensor 74 is higher than the level set by the adjusting pot 82a, the no pressure light 84 will go OFF.

In a high flow rate mode, the ON switch 12 will be in the high light 86 position. The first relay 16 is energized and the contacts will switch to the second and fourth contacts 16b, 16d, respectively. The second contact 16b will put a ground to the second solenoid valve 20, and remove the ground from first solenoid valve 18 so that when the smoke evacuator is activated, the second solenoid valve 20 will open the flow on the first tubing 22 and will close the flow on the third tubing 44. The first solenoid valve 18 will also stay closed because the ground is removed. The adjusted flow rate will be much higher and this will be reflected on the bar graph 26 which is now energized from fourth contact 16d of first relay 16.

The fourth relay 52 will change contacts to (H) so it will read the flow rate through the F/V converter 88. This invention presents two ways to read the flow rate. One way is to read the flow rate through the flow meter 46 and the other way is to read the flow rate by reading the frequency delivered by rotation of the pump. Both methods can be used either simultaneously or separately. This will not depart from the purpose of the present invention.

The differences described above with respect to the high flow rate mode are the only differences between the high flow rate mode of the present invention for open surgical procedures and the low flow rate mode of the present invention for laparoscopic surgical procedures. Everything else is identical in both modes and therefore will not be repeated. The one exception is that the pressure sensor 74 will not be used in open surgical procedures since there is no abdominal pressure which must be maintained for open surgical procedures.

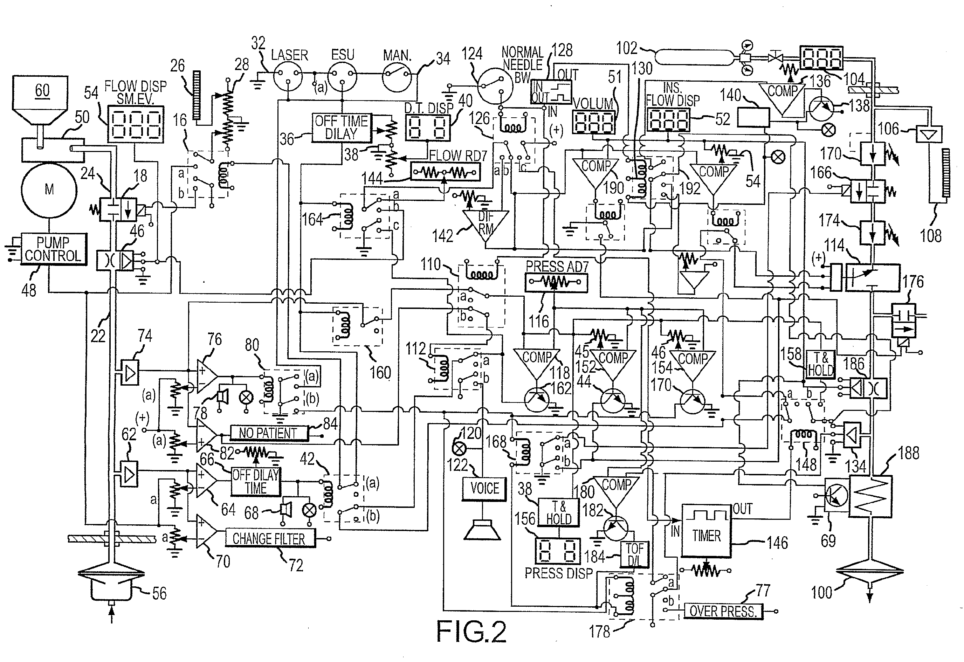

Figure 2 depicts an automatic smoke evacuator system and insufflation and apparatus having an insufflation means in accordance with the present invention which enables a patient's intra-abdominal pressure to be maintained throughout laparascopic surgical procedures.

The smoke evacuator and insufflation system and apparatus portion of the second embodiment is identical with the one described in Figure 1 in relation to the first embodiment. Therefore, the detailed description of the second embodiment which related to the smoke evacuator and insufflation system and apparatus will not be repeated; instead, only the system and apparatus relating to the insufflation means will be described. The set-up and function of the entire apparatus when used with a patient for laparoscopic surgical procedures will be described.

The patient is connected for the smoke evacuation through the tubing at the filter 56 as mentioned above. The smoke evacuator side set-up and function of the second embodiment shown in Fig. 2 is identical with the one described in Figure 1. Turning now to the insufflation side, the patient is attached through the tubing at the second filter 100 for insufflation. The gas tank 102 is installed and the valve is open so the high pressure side is activated. The third filter 104 will filter the gas on the high side so that no debris will enter the apparatus and be transferred into the patient. The insufflation pressure sensor 106 will read the gas tank pressure and will show the volume of gas in the tank 102 on volume display 108.

At first, the insufflation is performed through a needle in order to safely prepare the patient for introducing a working CANULA. At this time, the smoke evacuator side will not be attached because there is no place to attach it to. Therefore, the vacuum pressure sensor 74 cannot read any pressure and cannot be used for the initial insufflation.

If the operator attempts to use the insufflator in the normal mode for the needle, the zero pressure at the vacuum pressure sensor 74 will activate the no pressure light 84 through the fourth comparator 82. At the same time, a signal is sent through the normally closed contact 110b of a sixth relay 110 to a seventh relay 112 which will activate the normally closed contact 112a. The proportional solenoid 114 will not open because the signal sent by the pressure adjust 116 through the fifth comparator 118 will he interrupted. The normally open contact 112b will close and a signal will be sent to a visual warning 120 and an audible alarm 122, which can be a voice message warning the personnel to switch to a needle position. This safety factor is necessary to ensure that no excessive pressure conditions result since the active pressure sensor 74 is not connected to the patient.

During correct use, the insufflation switch 124 will be switched to the needle position. This will activate an eighth relay 126 and the sixth relay 110 and at the same time the sixth relay 110 will open contact 110b, which is normally closed, and the no pressure signal 84 from the pressure sensor 74 can not reach the seventh relay 112, so no alarm condition will be read. At the same time, a power-up once shot 128 is activated and a simple pulse signal is sent to a latching relay 130 which will close contact 130a, which is normally open, and will switch from contact 130b to 130c on the other contacts. The OP AMP 132 will send a signal through contact 130c of latching relay 130 to the proportional solenoid valve 114 so that a very small flow of gas is released. This low flow rate will read a minimal resistance in line but as long as the needle is not in the intra-abdominal cavity, it will show an occlusion and a pressure sensor 134 will read it. The signal will reach a sixth comparator 136 through contacts in the latching relay 130 which are now closed. The sixth comparator 136 will send a signal through a transistor 138 to a special timer 140 which will energize but not activate. At the same time, a visual sign will indicate that the needle has penetrated the abdominal wall but has not reached the intra-abdominal cavity for a safe insufflation. As the needle is pushed forward, the needle will reach the intra-abdominal cavity. The occlusion condition is eliminated and the signal of the input from the timer 140 will stop. The warning light will go off and the timer 140 will activate with a pulse delivered at the other side of the latching relay 130, switching the contact 130a to open and contact 130c to contact 130b. This will establish the connection between a differential amplifier 142 and the proportional solenoid 114 for needle insufflation. This flow rate will be much higher than the previous flow rate but smaller than the maximum flow rate in normally functioning conditions.

As mentioned previously, when insufflator switch 124 is in the needle position, the eighth relay 126 is activated. Therefore, contact 126b will be switched to 126a and the connection between flow adjust 144 and the proportional solenoid 114 is made through the differential amplifier 142 so the maximum flow rate will be much lower than normal conditions. Also, contact 126c, which is normally open, will close sending a signal to pulse timer 146 which in turn will send an alternative ON and OFF signal to a ninth relay 148 which will alternate open and closed between normally closed contact 148a and normally open contact 148b.

When contact 148a is closed, the signal from the fifth comparator 118 (which will send a signal as long as the pressure is below the pressure adjusted by pressure adjust 116) will send a signal through the normally closed contacts 112a, 42b and a tenth relay 150, to the ON/OFF of the proportional solenoid 114 turning it ON. When contact 148a is open, the proportional solenoid 114 will shut OFF allowing the pressure sensor 134 to measure the intra-abdominal pressure. When contact 148a is open, contact 148b is closed which will send the pressure reading to the fifth comparator 118 for controlling the pressure and the seventh and eighth comparators 152 and 154, respectively, for safety, and also to a pressure display 156. When the contact 148b is open, the threshold 158 will maintain the last reading before the contact 148b was opened.

When the peritoneum pressure reaches the desired pressure, the canulas are installed and the smoke evacuator tubing is also attached. The pressure sensor 74 is activated and the "no patient" light 84 will turn OFF.

The insufflator switch 124 will be switched to "normal" and the sixth relay 110 and the eighth relay 126 will deactivate and the position of the contacts is as shown in Figure 2.

The desired pressure is adjusted by pressure adjust 116. The signal will go to the reference on the fifth comparator 118. The signal will also go to the reference on the seventh and eighth safety comparators 152 and 154. Under normal conditions, the active side of the comparator is coming from the pressure sensor 74 through the contacts of the eleventh relay 160 which are normally closed and the contacts of the sixth relay 110 which are normally closed. When the pressure at pressure sensor 74 is lower than the pressure set by pressure adjust 116, the fifth comparator 118 will send a signal to the base of transistor 162 which will put a ground on the collector. The ground signal will pass through contacts 112a of the seventh relay 112, which are normally closed, then through the normally closed contacts 42b of the third relay 42, then through the contacts 148a of the ninth relay 148, which are normally closed, and finally through the contacts of the tenth relay 150, which are normally closed, which will open the proportional solenoid valve 114. The flow which is going to be delivered is determined by the same proportional solenoid valve 114 by receiving a proportional signal from flow adjust 144 through the normally closed contact 164a of the twelfth relay 164, through the contact 126b of the eighth relay 126, and then through the contact 130b of the latching relay 130. At the same time, since no fault condition is existing, the solenoid 166 is also energized open by the ground from relay contact 168a which is normally closed. This normally open state of the solenoid 166 is also for safety reasons, so that if energy is inadvertently lost, the normal position of the solenoid 166 is closed. This safety feature is called "fail safely." The foregoing describes how the insufflation means of the present invention works to provide gas flow to the patient.

Once the gas flow to the patient has started, it will pass through a precise pressure regulator 170 which will drop the pressure to the desired working pressure. As previously indicated, the solenoid 166 is normally closed, but when the insufflator is ON and the conditions are normal, the solenoid 166 is energized. The solenoid 166 will be turned OFF if the pressure exceeds the adjusted pressure and the eighth comparator 154 will activate the transistor 172, which will in turn open the contact 168a of the thirteenth relay 168 when the ground is removed and the solenoid 166 is closed. The solenoid 166 will also close if the pressure sensor 74 registers high pressure and the eighth comparator 154 fails to activate. Then, the third comparator 76 will energize the contact 80b of the fifth relay 80 which in turn will energize the thirteenth relay 168. Contact 168a is open thereby removing the ground from the solenoid 166 which will close.

The flow will pass from the solenoid 166 through a mechanical pressure release valve which will release the flow outside if the pressure exceeds the pressure adjusted at the pressure regulator 170. From solenoid 174, the flow will enter the proportional valve 114, whose function has been previously described above.

Next, an exhaust valve 176 will open if the pressure exceeds the adjusted pressure at the pressure sensor 74 and the seventh comparator 152. This first safety feature is designed to balance the pressure by releasing some of the gas when the pressure slightly exceeds the adjusted pressure.

If the intra-abdominal pressure is substantially higher than the adjusted pressure/ then the eighth comparator 154 will activate the thirteenth relay 168 and the solenoid 166 will close. The exhaust valve 176 will then open releasing the gas and the proportional solenoid 114 will be closed.

If the intra-abdominal pressure reaches unsafe limits, the third comparator 176 will activate the contacts 80a, 80b of the fifth relay 80. Further, the smoke evacuator will be activated by closing contact 80a and activating the off time delay 36 so the gas will be forcibly removed from the abdominal cavity.

When the latching relay 130 is activated, latching relay 178 is also activated. Both relays need only a pulse signal for activation and will maintain the contact in position until another signal is delivered that will deactivate the relay. In this case/ both relays 130 and 178 are activated at the same time and the contact 178b of the latching relay 178 will turn ON a visual and audio signal for excessive pressure, and contact 178a will introduce the pressure sensor 134 into the circuit. Because there is no gas flow on the insufflator side at this time, the pressure sensor 74 cannot be used while the smoke evacuator is being used to remove excess pressure.

The pressure sensor 134 will monitor the excessive pressure condition by sending a signal through the latching relay contact 178a, which is activated so that the contacts 178a, 178b are closed, and to the ninth comparator 180 as the active signal. The reference signal comes from the pressure adjust 116. So, as the pressure drops and reaches the adjusted level, the ninth comparator 180 will activate the transistor 182 and a time off delay 184 for maintaining the signal flow for a predetermined number of seconds after the latching relay 178 is deactivated and contact 178a opens so that no signal from the pressure sensor 134 will be available.

At the same time, thirteenth relay 168 is deactivated and everything approaches normal. The gas flow passes through the flow meter 186, through the pressure sensor 134 and into the gas wanner 188. The temperature of the gas is determined by the signal received from the flow meter 186. The higher the flow rate, the higher the temperature, so it will maintain the same temperature as the patient. From the gas warmer 188, the gas travels through the filter 100 and to the patient.

The functioning of the insufflator described above relates to both normal and excessive pressure conditions where the smoke evacuator side is only used for pressure readings and for the safety of actively turning on the smoke evacuator when the pressure is too high.

Assume that a patient's intra-abdominal cavity is at normal pressure and that a laparoscopic surgical procedure is about to begin. When a laser or ESU is activated for laparoscopic surgery, the sensor plugged in at the connector 32 will turn on the smoke evacuator via the off time delay 36. The flow is adjusted by the flow adjusting pot 28. When the smoke evacuator is activated, the eleventh and twelfth relays 160 and 164 are also activated.

Contact 164a of the twelfth relay 164 that comes from INSUFFLATOR FLOW adjust 144 will switch to contact 164b and the signal coming from the smoke evacuator flow meter 46 will adjust the proportional solenoid 114 to exactly the same flow rate as the smoke evacuator. At the same time, a signal from flow meter 46 and flow meter 186 are compared by a tenth comparator 190 which will turn the exhaust valve 176 ON if the insufflator flow is slightly higher than the smoke evacuator flow. If the flow of the insufflator is substantially higher than the smoke evacuator flow, an eleventh comparator 192 will also shut off the proportional solenoid 114. The contact 164c of the twelfth relay 164 will turn ON the proportional solenoid 114 by delivering the ground to the normally closed contact 112a of the seventh relay 112, and the same way as described in with reference to a normal function.

The eleventh relay 160 will open the contact so the pressure sensor 74 is disconnected from the insufflator since it can not read the pressure when the smoke evacuator is activated. When an occlusion condition at the smoke evacuation is present, the vacuum sensor 62 will send the signal at the first comparator 64 and from comparator 64, the off time delay 66 is activated which in turn will activate the third relay 42. The contact 42a will open thereby stopping the smoke evacuator pump, and contact 42b will open thereby deactivating the proportional solenoid 114 by removing the ground. At the same time, the occlusion visual and audio alarm 68 will turn on.

## Claims

1. An automatic smoke evacuation and insufflation apparatus (10) comprising:
a vacuum pump (50) for removing at least one of a gas, a smoke and debris from a surgical site;
a vacuum sensor (62) for sensing a vacuum level generated by the vacuum pump;
an insufflator for supplying a gas to a body cavity of a patient to maintain a desired pressure within the body cavity;
a flow meter (46,186) for measuring a flow of the gas from the insufflator;
**characterized by**
a gas warmer (188) adapted to increase the gas to a higher temperature when the gas flow from the insufflator is high and to reduce the gas to a lower temperature when the gas flow from the insufflator is low.

2. The apparatus of claim 1 further comprising at least one filter (60) connected to the vacuum pump (50).

3. The apparatus of claim 1 further comprising at least one filter (56) connected to the insufflator.

4. The apparatus of claim 1 wherein the vacuum sensor (62) is capable of shutting off the vacuum pump (50) when the vacuum level is lower than its set level.

5. The apparatus of claim 1 further comprising a pressure sensor (74) for sensing an actual pressure within the body cavity.

6. The apparatus of claim 5 wherein the pressure sensor (74) is capable of activating the vacuum pump (50) if the actual pressure is too high so that gas is removed from the body cavity until the actual pressure returns to a normal level.

7. The apparatus of claim 5 further comprising at least one valve (18,20,114) for shutting off the insufflator.

8. The apparatus of claim 1 further comprising at least one of an audio alarm (78,122) and a visual alarm (68,77,120) which is activated if a pressure within the body deviates from the desired pressure.

## Patentansprüche

1. Automatische Rauchabzugs- und Insufflationsvorrichtung (10), umfassend:
eine Vakuumpumpe (50) zum Entfernen von mindestens einem von einem Gas, einem Rauch und Schmutz aus einer Operationsstelle;
einen Vakuumsensor (62) zum Erfassen eines von der Vakuumpumpe erzeugten Vakuumniveaus;
einen Insufflator zum Zuführen eines Gases zu einer Körperhöhle eines Patienten, um einen gewünschten Druck innerhalb der Körperhöhle aufrechtzuerhalten;
einen Durchflussmesser (46, 186) zum Messen eines Durchflusses des Gases aus dem Insufflator;
**gekennzeichnet durch**
einen Gaswärmer (188), der dazu eingerichtet ist, das Gas auf eine höhere Temperatur zu erhöhen, wenn der Gasdurchfluss von dem Insufflator hoch ist, und das Gas auf eine niedrigere Temperatur zu reduzieren, wenn der Gasdurchfluss von dem Insufflator niedrig ist.

2. Vorrichtung nach Anspruch 1, ferner umfassend mindestens einen Filter (60), der mit der Vakuumpumpe (50) verbunden ist.

3. Vorrichtung nach Anspruch 1, ferner umfassend mindestens einen Filter (56), der mit dem Insufflator verbunden ist.

4. Vorrichtung nach Anspruch 1, wobei der Vakuumsensor (62) in der Lage ist, die Vakuumpumpe (50) abzuschalten, wenn das Vakuumniveau niedriger als sein eingestelltes Niveau ist.

5. Vorrichtung nach Anspruch 1, ferner umfassend einen Drucksensor (74) zum Erfassen eines Ist-Drucks innerhalb der Körperhöhle.

6. Vorrichtung nach Anspruch 5, wobei der Drucksensor (74) in der Lage ist, die Vakuumpumpe (50) zu aktivieren, wenn der Ist-Druck zu hoch ist, sodass Gas aus der Körperhöhle entfernt wird, bis der Ist-Druck auf ein normales Niveau zurückkehrt.

7. Vorrichtung nach Anspruch 5, ferner umfassend mindestens ein Ventil (18, 20, 114) zum Abschalten des Insufflators.

8. Vorrichtung nach Anspruch 1, ferner umfassend mindestens einen von einem Audioalarm (78, 122) und einem visuellen Alarm (68, 77, 120), der aktiviert wird, wenn ein Druck innerhalb des Körpers von dem gewünschten Druck abweicht.

## Revendications

1. Appareil d'insufflation et d'évacuation automatique de fumée (10) comprenant :
une pompe à vide (50) pour retirer d'un site chirurgical au moins l'un parmi un gaz, une fumée et des débris ;
un capteur de vide (62) pour détecter un niveau de vide généré par la pompe à vide ;
un insufflateur pour distribuer un gaz à une cavité corporelle d'un patient afin de maintenir une pression souhaitée à l'intérieur de la cavité corporelle ;
un débitmètre (46, 186) pour mesurer un débit du gaz provenant de l'insufflateur ;
**caractérisé par**
un réchauffeur de gaz (188) adapté pour porter le gaz à une température plus élevée lorsque le débit de gaz provenant de l'insufflateur est élevé et pour amener le gaz à une température plus basse lorsque le débit de gaz provenant de l'insufflateur est faible.

2. Appareil selon la revendication 1, comprenant en outre au moins un filtre (60) relié à la pompe à vide (50).

3. Appareil selon la revendication 1, comprenant en outre au moins un filtre (56) relié à l'insufflateur.

4. Appareil selon la revendication 1, dans lequel le capteur de vide (62) est capable d'arrêter la pompe à vide (50) lorsque le niveau de vide est inférieur à son niveau de consigne.

5. Appareil selon la revendication 1, comprenant en outre un capteur de pression (74) pour détecter une pression réelle à l'intérieur de la cavité corporelle.

6. Appareil selon la revendication 5, dans lequel le capteur de pression (74) est capable d'activer la pompe à vide (50) si la pression réelle est trop élevée, de telle sorte que du gaz est retiré de la cavité corporelle jusqu'à ce que la pression réelle retourne à un niveau normal.

7. Appareil selon la revendication 5, comprenant en outre au moins une valve (18, 20, 114) pour arrêter l'insufflateur.

8. Appareil selon la revendication 1, comprenant en outre au moins une parmi une alarme audio (78, 122) et une alarme visuelle (68, 77, 120) qui est activée si une pression à l'intérieur du corps s'écarte de la pression souhaitée.
